# EUROPEAN PATENT SPECIFICATION

(11) **EP 3 357 444 B1**
(45) Date of publication and mention of the grant of the patent: **04.12.2019**
(21) Application number: 17154861.3
(22) Date of filing: 06.02.2017
(51) Int. Cl.: A61B 17/86, A61F 2/00, A61B 50/30

(54) **PACKAGING ASSEMBLY**
VERPACKUNGSANORDNUNG
ENSEMBLE DE CONDITIONNEMENT

(43) Date of publication of application: 08.08.2018
(73) Proprietor: Synthes GmbH, 4436 Oberdorf (CH)
(72) Inventor: GRABOWSKI, Woitech, 4410 Liestal (CH); SCHÜLE, Alexander, 4500 Solothurn (CH)
(74) Representative: Klunker IP Patentanwälte PartG mbB

(56) References cited:
- US-A- 6 006 917
- US-A- 6 161 695
- US-B1- 6 889 839

## Description

### BACKGROUND

This invention relates to a packaging assembly for a medical device as well as a method of packaging a medical device.

Medical devices, such as bone implants, for example bone plates, intramedullary devices, artificial joints, or the like, are packaged in a controlled environment, in particular a clean room, and then sterilized before shipping to a customer, such as a hospital. Typically, a double sterile barrier system may be used to seal the medical device against the environment. The double sterile barrier may comprise two pouches. The medical device is inserted into a first, inner pouch, which is vacuumed and sealed. While still in the controlled environment, the sealed first pouch is then inserted into a second, outer pouch, which in turn is also vacuumed and sealed. Likewise, more than two pouches or only a single pouch may be used as a sterile barrier. The pouches with the medical device are then inserted into a secondary packaging, such as a cardboard box or the like, for shipping and transport and then sterilized.

The sterile barrier must remain intact in order to avoid contamination of the medical device. However, the sterile barrier, which comprises one or more, usually two, pouches, is dimensionally unstable. This means that, in particular during transport, the pouches with the medical device may move within the secondary packaging. The pouches being vacuumed may reduce movement of the medical device inside the pouch. However, in particular large and heavy products may damage the pouches if they move in the secondary packaging and hit the inner walls of the secondary packaging. Thus, additional protective components are required, such as foams or other cushioning materials, which contribute to the overall costs and complexity of the packaging system.

Other issues may arise with dimensionally unstable packaging assemblies during handling. The pouches must be correctly placed in the secondary packaging along with protective components or cushioning material. Insertion of the sealed first pouch with the medical device into the second pouch may be difficult and cumbersome because the pouches may buckle or crumple.

US 6,161,695 discloses a packaging system for a medical device in which the medical device is first placed in a support tray, which has a recessed area sized and shaped for receiving the medical device. The support tray with the medical device is then inserted into a first pouch, which is evacuated and sealed. The first pouch is then inserted into a second pouch, which is also evacuated and sealed. The pouches with the medical device are put into a box for transport. To make the pouches fit into the box, edges of the pouches may be folded.

Another packaging system for a medical device is disclosed in US 6,006,917. The medical device is placed in a first pouch, which is evacuated and sealed. The first pouch is inserted into a second pouch, the inner dimensions of which correspond in size and shape to the outer dimensions of the first pouch to hold the first pouch in place. The second pouch is clamped in a frame such that the medical device is suspended in the frame and does not contact inner surfaces of a transport box.

### SUMMARY OF THE INVENTION

It is an object of the present invention to provide a packaging assembly for a medical device as defined in claim 1, and a method of packaging a medical device, as defined in claim 8, which provide secure protection for the medical device at reduced packaging costs and reduced complexity. Preferred embodiments are defined in the dependent claims.

A packaging assembly for a medical device comprises at least one pouch, in particular at least two pouches, such as a first pouch and a second pouch. The pouches are sealed in order to provide a sterile barrier between the environment and the medical device received in the pouches. In particular, the medical device may be received in the first pouch, which in turn may be received in the second pouch. Thus, the first pouch can be regarded as an inner pouch and the second pouch can be regarded as an outer pouch. Accordingly, in a method of packaging the medical device, the medical device is inserted into the at least one pouch. In particular, the medical device may be first inserted into the first pouch, which is then sealed to provide a sterile barrier between the medical device and the environment. The sealed first pouch containing the medical device may then be inserted into the second pouch. The pouches might also be formed as envelopes, bags, or other wrappers.

The packaging assembly further comprises at least one support member, wherein, in an assembled configuration, at least the first pouch and the support member are secured against movement relative to each other by at least one of the first and second pouches being folded around the support member. In particular, folding the pouches around the support member may happen either before or after inserting the first pouch with the medical device into the second pouch.

According to the invention, the first pouch, preferably the first and second pouches, are secured relative to the support member only by folding. That means no additional attachment, in particular no permanent attachment, such as adhesive, stapling, taping, welding, or the like, is necessary for securing the relative positions of the first pouch and the support member. It will be appreciated, that also the relative positions of the second pouch and the support member may be secured, in particular if the first and second pouches are secured relative to each other, e.g. by a vacuum in the second pouch.

The packaging assembly including the support member provides dimensional stability. This has the effect that less or no additional cushioning material or protective components are required within a secondary packaging because the support member prevents the medical device from damaging the pouches. Packaging costs can be reduced. This also reduces waste because less packaging material is used. Further, the packaging components are reduced and therefore the risk of human error during the packaging process. The support member engages the at least one pouch, in particular the first and second pouches, by folding such that no additional fixation means are required.

The support member may be disposed inside the at least one pouch or outside the at least one pouch. In particular, if the packaging assembly comprises at least two pouches, such as a first, inner pouch and a second, outer pouch, the support member may be disposed inside the second pouch and outside the first pouch, or more generally may be disposed between adjacent pouches of the at least two pouches. Alternatively, the support member may be disposed inside the innermost pouch along with the medical device, or the support member may be disposed outside the outermost pouch. Apart from the position of the support member relative to the at least one pouch, the support member may be folded or may be left unfolded as will be described in more detail below.

In a first embodiment, the support member may be disposed outside the first pouch inside the second pouch. This means that folding the first pouch around the support member is performed before inserting the first pouch containing the medical device into the second pouch, and that the support member along with the first pouch containing the medical device is inserted into the second pouch. Thus, the support member is placed inside the double sterile barrier between the first and second pouches. Since the first pouch is inserted into the second pouch, which may fit closely around the first pouch when supported by the support member, the insertion process is facilitated because the support member may act as an insertion aid for the first pouch.

Independent from the number of pouches and the position of the support member, but preferably in the context of the aforementioned first embodiment, the support member may be sized and shaped to support the at least one pouch, in particular the first pouch, such that at least a portion of a rim region of the first pouch extends beyond the support member. Said portion of the rim region is then folded around the support member. In particular, the first pouch may have a substantially rectangular shape, and said portion of the rim region of the pouch that is to be folded may comprise at least one edge of the pouch, preferably at least two opposing edge portions of the pouch. For instance, the opposing narrow edges of the pouch may extend beyond the support member in a longitudinal direction, such that the narrow edges can be folded around the support member. Alternatively or in addition, the longitudinal edges may extend beyond the support member in a transverse direction such that they also can be folded around the support member. Preferably, only two opposing edges of the pouch extend beyond the rim region of the support member to avoid cross folds in the pouch, which could create weak points in the pouch.

Preferably, the position of said folded portion of the rim region of the first pouch is secured by a vacuum in the second pouch. In particular, it is preferably not permanently attached to the support member, e.g. by adhesive, welding, or the like. The folded first pouch is held in place only by means of engagement with the interior of the second pouch. It will be appreciated that a vacuum in the second pouch is not absolutely necessary for holding the first pouch. For example, if the second pouch fits closely enough around the first pouch and the support member, the folded edges of the first pouch will also stay in place.

Finally, in particular before inserting the packaging assembly into a secondary packaging, at least a portion of a rim region of the second pouch, preferably at least two opposing edge portions of the second pouch, may be folded around the support member with the folded first pouch. It will be appreciated that in this embodiment the support member is preferably not folded. It may be made of a plastics material. For example, the support member may be a sheet of PETG (polyethylene terephthalate glycol-modified). Generally, the support member may have a substantially planar body. The material or the thickness of the planar body, or both, are chosen such that the support member is stiff enough to provide the desired protection. In order to provide additional stiffness, the support member may be structured, e.g. by providing a honeycomb structure, zig-zag profile or other structures suitable to increase the stiffness of the support member.

In a second embodiment, the support member is disposed outside the at least one pouch, such as outside the second pouch, generally outside the outermost pouch. Thus, the second pouch containing the first pouch containing the medical device is placed on the support member after the second pouch is sealed. Folding of the pouches around the support member is carried out after the pouches are placed on the support member. In contrast to the first embodiment, the support member is placed outside the sterile barrier in the second embodiment. Furthermore, in the second embodiment the support member is also folded as will be described in more detail below, whereas in the first embodiment the support member is not folded.

Independent from the number of pouches and the position of the support member, but preferably in the context of the aforementioned second embodiment, the support member may be sized and shaped to support the at least one pouch, preferably the second pouch, such that at least a portion of a rim region of the second pouch, preferably at least two opposing edge portions of the second pouch, extends into or beyond a rim region of the support member. The rim region of the support member may be defined by at least one predetermined fold line, which may be defined e.g. by creases. This enables the at least one pouch, preferably the second pouch, to be folded along with the support member, which improves engagement of the pouch with the support member after folding. Preferably, only two opposing edges of the pouch extend into or beyond the rim region of the support member to avoid cross folds in the pouch, which could create weak points in the pouch.

At least a first portion of the rim region of the support member, preferably at least two opposing edge portions of the support member, is folded along the predetermined fold line. In an assembled configuration, the folded first portion of the rim region may extend at an angle relative to the central portion of the support member in order to define a receptacle for the medical device. This may be achieved by a biasing force that urges the folded first portion of the rim region of the support member, e.g. the opposing longitudinal edges, back to their unfolded configuration. Further, corners of the support member may define angled fold lines that allow the receptacle to be formed in the folded configuration. In particular, the fold lines may be arranged such that a height of the receptacle, i.e. the distance between the central portion of the support member and the folded first portion of the rim region, may increase towards the center of the support member in a longitudinal direction.

In the further course of the packaging process, at least a second portion of the rim region of the support member, preferably at least two opposing edge portions of the support member, is folded along the predetermined fold line. Preferably, the at least one pouch, more specifically at least the second pouch, is folded along with the support member in order to secure the second pouch relative to the support member. It will be appreciated that the first pouch, which is received in the second pouch, may also be folded along with the second pouch and the support member if the first pouch also extends into or beyond the rim region of the support member. If the first pouch is securely held within the second pouch, e.g. by vacuum, the position of the first pouch relative to the support member is secured by securing the position of the second pouch relative to the support member. It may also be possible for none of the pouches to be folded along with the support member, but rather for the support member to be folded around the pouches in order to avoid relative movement of the pouches and the support member.

In some cases, in an assembled configuration, the folded rim region of the support member may enclose an angle with a central portion of the support member. In other words, the support member, possibly along with at least the second pouch, might not be folded completely by 180 degrees to abut against itself due to the resiliency in the material. The characteristics of the material of the support member and possibly also the pouches that are folded together with the support member may create a biasing force that urges the support member towards its original planar configuration such that the folded rim region extends at an angle relative to the central portion. This "incomplete" folding may be advantageous because it increases the transverse dimension of the assembled packaging assembly, which may secure the packaging assembly within a secondary packaging in a transverse direction. However, generally in order to provide a compact packing assembly, it may be desirable to fold the rim region "completely", i.e. 180°, with respect to the central portion to abut the central portion.

The second portion of the rim region of the support member, which is different from the aforementioned first portion, may be folded along a predetermined fold line in an opposing direction with respect to the first portion of the rim region of the support member. For example, the aforementioned first portion of the rim region of the support member may comprise two opposing longitudinal edges of a rectangular pouch, whereas the second portion of the rim region may comprise two opposing narrow edges. For instance, first the two opposing longitudinal edges can be folded upwards onto a surface that carries the pouches with the medical device, so as to extend partly over the pouches. After that, the two opposing narrow edges may be folded downwards along with at least the second pouch to secure the pouches with respect to the support member. This folding technique allows simple and efficient fixation of the two pouches with the medical device to the support member. All portions of the rim region that are to be folded can be defined by predetermined fold lines, which may be formed e.g. by creases. As the person skilled in the art will understand, predetermined fold lines can be created in cardboard for example by impression or perforation.

Preferably, the support member has a substantially planar body, at least in an unassembled configuration. The planar body can then be folded along predetermined fold lines after the pouches have been placed on top of the support member. The support member may be made of cardboard. It will be appreciated that other materials, such as plastics materials as described above with respect to the first embodiment, can be chosen for the support member of the second embodiment to provide sufficient stability. Since the support member is placed outside the sterile barrier in the second embodiment, the material of the support member does not have to fulfill sterile or biocompatible requirements. This has the further advantage that the packaging is more sustainable as no additional plastic components are required.

Now referring to both embodiments, a packaging system for a medical device may comprise any of the aforementioned packaging assemblies and a secondary packaging, such as a cardboard box, envelope, or the like. The secondary packaging may be sized and shaped to receive the packaging assembly, wherein inner dimensions of the secondary packaging correspond to outer dimensions of the packaging assembly in order to secure the packaging assembly against relative movement with respect to the secondary packaging. Since the dimensions of the packaging assembly match the dimensions of the secondary packaging, the packaging assembly carrying the medical device will be prevented from moving in the secondary packaging without the need for further cushioning material or protective components. In particular, at least the dimensions in a plane of the support member, i.e. length and width, may be chosen for a close fit into the secondary packaging in order to secure the packaging assembly in the secondary packaging. However, it may be advantageous if transverse dimensions of the packaging assembly are also chosen for a close fit into the secondary packaging, e.g. by adapting the folded edges of the second pouch or the support member, to further avoid transverse movement.

### BRIEF DESCRIPTION OF THE DRAWINGS

The foregoing summary, as well as the following detailed description of preferred embodiments, will be better understood when read in conjunction with the appended drawings. For the purpose of illustrating the present disclosure, reference is made to the drawings. The scope of the disclosure is not limited, however, to the specific embodiments disclosed in the drawings. In the drawings:
Figs. 1A to 1H show views of a packaging assembly according to a first embodiment during different stages of a packaging method according to a first embodiment; and
Figs. 2A to 2F show views of a packaging assembly according to a second embodiment during different stages of a packaging method according to a second embodiment.

### DETAILED DESCRIPTION

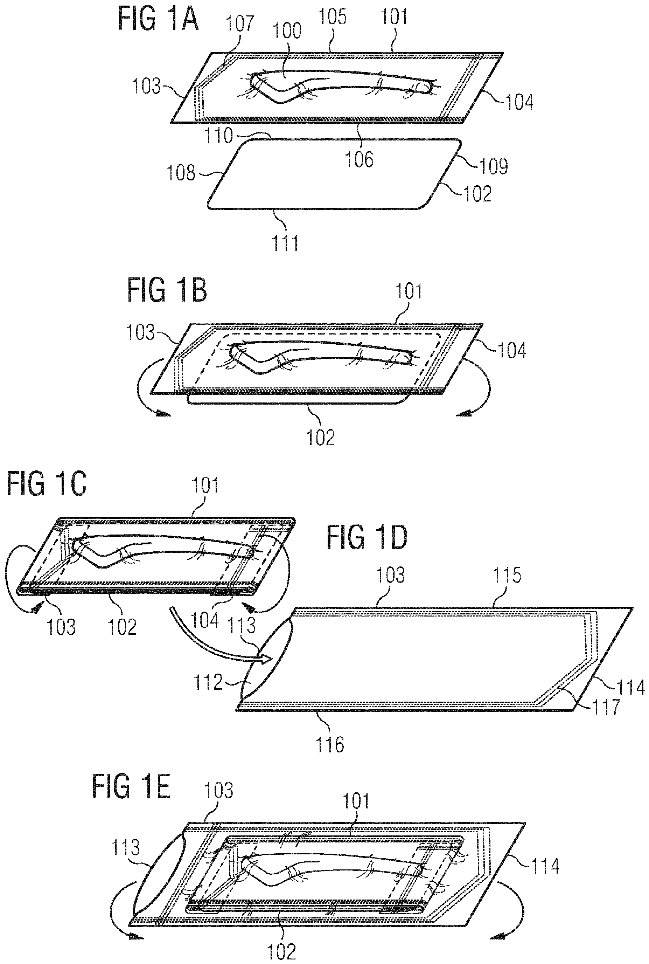

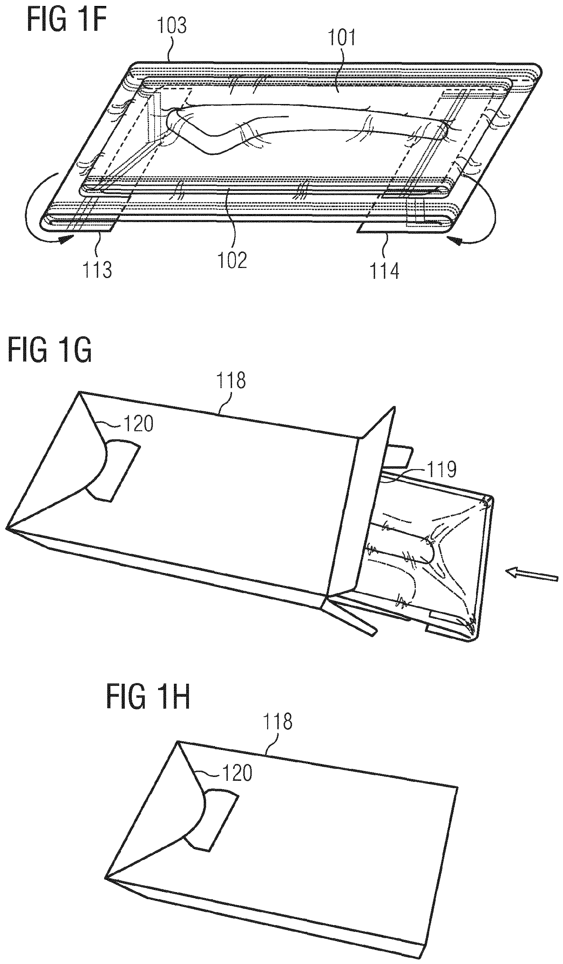

Referring to Figs. 1A to 1H, a packaging assembly according to a first embodiment and respective stages of a method of packaging a medical device are illustrated. A medical device 100, such as an intramedullary femoral part of an artificial hip joint, is provided in a first pouch 101. However, the medical device 100 may be any other bone implant, plate, or the like. The medical device 100 is inserted into the first pouch 101, which is then vacuumed and sealed along a seam 107 which allows the first pouch 101 to be opened by tearing or peeling the seam 107. The first pouch 101 has a substantially rectangular shape, with first and second opposing longitudinal edges 105, 106 and first and second opposing narrow edges 103, 104. It will be appreciated that the first pouch 101 may be closed at three of the edges before insertion of the medical device 100 by means of the seam 107, and the seam 107 is closed at the forth edge after insertion of the medical device 100 (here the edge 104). As shown in Fig. 1A, a support member 102 is provided. In this embodiment, the support member 102 is a sheet of plastics material, preferably PETG. Any other material fulfilling the requirements of sterile barrier components may be chosen. The support member 102 has a substantially rectangular shape, with first and second opposing longitudinal edges 110, 111 and first and second opposing narrow edges 108, 109. The length of the first pouch 101 is greater than the length of the support member 102, while the width of the first pouch 101 is substantially equal to the width of the support member 102. The first pouch 101, like any of the pouches described in the following, is preferably made of transparent plastic sheet or film. It will be appreciated that other materials, in particular nontransparent or opaque materials, can be chosen, such as nontransparent plastics or aluminum foil.

As indicated in Figs. 1B and 1C by arrows, the rim region of the first pouch 101 adjacent the opposing narrow edges 103, 104 is folded around the respective narrow edges 108, 109 of the support member 102. As shown in Fig. 1C, the first pouch 101 is folded closely around the support member 102, i.e. folds of the first pouch 101 substantially coincide with the narrow edges 108, 109 of the support member 102. Thus, the first pouch 101 is held relative to the support member 102.

In order to hold the folded configuration of the first pouch 101 in place, the first pouch 101 along with the support member 102 is inserted into a second pouch 103 as indicated in Fig. ID. The relatively stiff support member 102 may help to guide the first pouch 101 into the second pouch 103. Like the first pouch 101, the second pouch 103 is preferably made of transparent plastic sheet that is bonded along a seam 117 so as to allow the second pouch 103 to be opened by tearing or peeling the seam 117. The second pouch 103 has a substantially rectangular shape, with first and second opposing longitudinal edges 115, 116 and first and second opposing narrow edges 113, 114. An opening 112 is formed for insertion of the first pouch 101 along with the support member 102 and the medical device 100. The second pouch 103 is then vacuumed and sealed. The vacuum in the second pouch 103 improves fixation of the folded configuration of the first pouch 101 although the close fit in the second pouch 103 already holds the folded configuration of the first pouch 101 in place. The second pouch 103 has a size and shape suitable for receiving the first pouch 101.

The vacuumed and sealed second pouch 103 including the first pouch 101 with the medical device 100 and the support member 102 is shown in Fig. IE. Arrows indicate that the narrow edges 113, 114 of the second pouch 103 are folded. The final configuration of the packaging assembly is shown Fig. 1F. The folded edges 113, 114 of the second pouch 103 do not have to be secured to the bottom surface of the second pouch 103. They will be held in place by a secondary packaging 118 in which the packaging assembly is inserted as shown in Fig. 1G. The secondary packaging 118 has an opening 119 for insertion of the packaging assembly. The secondary packaging 118 may be opened by a customer by means of a tearing flap 120. It will be appreciated that the secondary packaging 118 may be any suitable packaging with other insertion openings and opening mechanisms. However, the size and shape of the secondary packaging 118, in particular its inner dimensions, correspond to the size and shape of the packaging assembly, in particular its outer dimensions. The loose folded edges 113, 114 may also contribute to fixation of the packaging assembly within the secondary packaging 118 without further cushioning material or protective components.

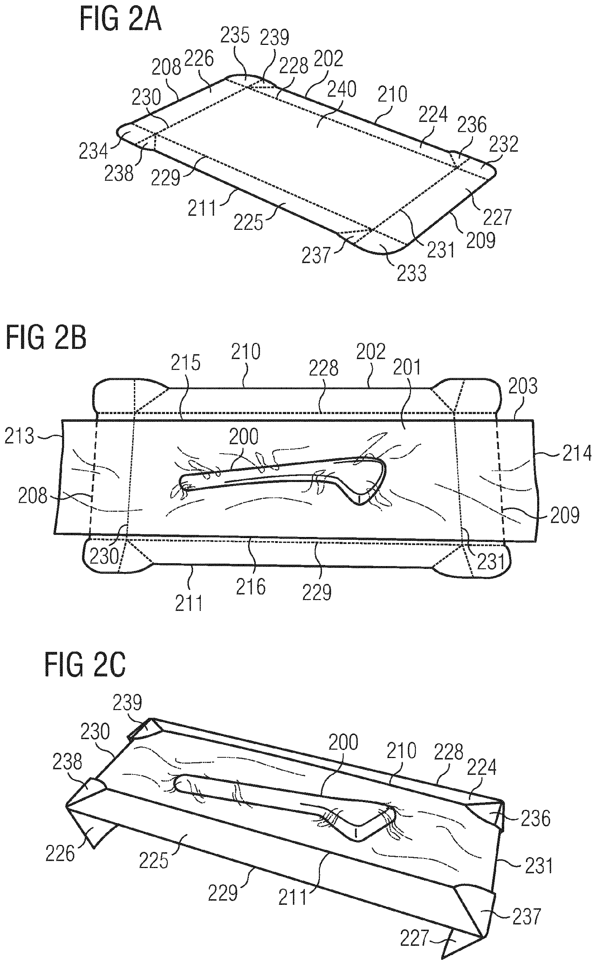

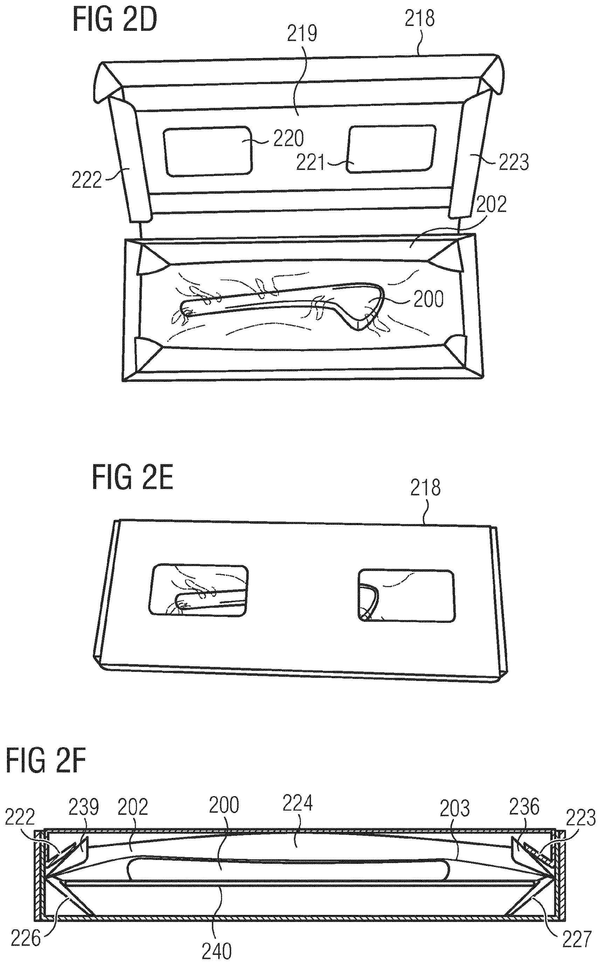

Referring now to Figs. 2A to 2F, a packaging assembly according to a second embodiment and respective stages of a method of packaging a medical device are illustrated. In contrast to the first embodiment of Figs. 1A to 1H, the packaging assembly of the second embodiment comprises a support member 202 that is disposed outside the sterile barrier, i.e. outside the pouches, as will be described in more detail below. The support member 202 is shown in an unassembled configuration in Fig. 2A. The support member 202 is formed of cardboard and has a substantially planar shape.

The support member 202 has a substantially rectangular shape, with first and second opposing longitudinal edges 210, 211 and first and second opposing narrow edges 208, 209. The support member 202 has a central body portion 240 that is bordered by predetermined fold lines 228, 229, 230, 231. The longitudinal fold lines 228, 229 define longitudinal portions 224, 225 of the rim region, while the transverse fold lines 230, 231 define transverse portions 226, 227 of the rim region. Corners 232, 233, 234, 235 are formed that may be regarded as belonging to both the longitudinal rim regions and the transverse rim regions. Connecting portions 236, 237, 238, 239 are formed by additional oblique fold lines. In this embodiment, the width of the support member 202 increases in a longitudinal direction towards the narrow edges 208, 209 (see Fig. 2B), which, however, is not absolutely necessary. The width may be constant along the entire length.

As shown in Fig. 2B, a medical device 200 is provided in a double sterile barrier formed of a vacuumed and sealed first, inner pouch 201 and a vacuumed and sealed second, outer pouch 203, similar to the first embodiment of Figs. 1A to 1H (in Fig. 2B the first pouch 201 is only indicated by its reference sign but not separately shown for the sake of simplicity). Vacuuming of the pouches 201, 203 is not absolutely necessary but advantageous for fixating the implant. The second pouch 203 has a substantially rectangular shape, with first and second opposing longitudinal edges 215, 216 and first and second opposing narrow edges 213, 214. The second pouch 203 extends beyond the narrow edges 208, 209 of the support member 202 in a longitudinal direction. However, a width of the second pouch 202 is smaller than a distance between the longitudinal fold lines 228, 229 of the support member 202. The first pouch 201 may extend beyond the fold lines 230, 231 or beyond the narrows edges 208, 209 of the support member 202 or may have a shorter length.

As illustrated in Fig. 2C, the support member 202 is folded along the fold lines 228, 229, 230, 231 in order secure the pouches 201, 203 containing the medical device 200 relative to the support member 202. In particular, in a first folding step, the longitudinal portions 224, 225 are folded towards a top surface of the support member 202, i.e. a surface carrying the pouches 201, 203, along the fold lines 228, 229 so that the folded portions 224, 225 extend partly over the pouches 201, 203. This creates a fixation of the pouches 201, 203 relative to the support member 202 in a transverse direction. Then, in a second folding step, the transverse portions 226, 227 of the support member 202 are folded in an opposing direction towards a bottom surface of the support member 202 in order to longitudinally secure the pouches 201, 203 relative to the support member 202. Due to its length, the second pouch 203 is folded together with the support member 202 in this second folding step. Also, the folded longitudinal portions 224, 225 engage the second pouch 203 so that the second pouch is easily folded along with the support member 202. As can be seen in the perspective view of Fig. 2C, the triangular connecting portions 236, 237, 238, 239 have the effect that the longitudinal portions 224, 225 do not abut against the top surface of the support member 202 but form a receptacle for the medical device 200. The transverse portions 226, 227 extend at an angle relative to the central portion 240 in the folded configuration.

The packaging assembly is then inserted into a secondary packaging 218, which may be in the form of a cardboard box. In order to visibly access the medical device 200 in the closed secondary packaging 218, the secondary packaging 218 has windows 220, 221 covered by a transparent plastic film 219. It will be appreciated that the windows 220, 221 can be omitted. The secondary packaging 218 is shown as a box that opens toward the top. It will be appreciated that any other suitable secondary packaging can be used. However, the size and shape of the secondary packaging 218, in particular its inner dimensions, correspond to the size and shape of the packaging assembly, in particular its outer dimensions. In particular the outer dimensions of the support member 202 secure the packaging assembly in the secondary packaging 218. The angled portions 226, 227 also contribute to fixation of the packaging assembly in a transverse direction towards the bottom within the secondary packaging 218 without further cushioning material, as can be seen in the side view of Fig. 2F. The angled portions 224, 225 prevent the packaging assembly from moving upwards within the secondary packaging 218. Further fixation is achieved by flaps 222, 223 extending from the lid of the secondary packaging 218.

It will be appreciated by those skilled in the art that changes may be made to the embodiments described above without departing from the inventive concept thereof. It should further be appreciated that structural features and methods associated with one of the embodiments can be incorporated into other embodiments. It is understood, therefore, that this invention is not limited to the particular embodiments disclosed, but rather modifications are also covered within the scope of the present invention as defined by the appended claims.

## Claims

1. A packaging assembly for a medical device (100; 200), comprising at least a first pouch (101; 201) and a second pouch (103; 203) sealed so as to provide a sterile barrier between the environment and the medical device (100; 200) received in the first pouch (101; 201), which in turn is received in the second pouch (103; 203), the packaging assembly further comprising at least one support member (102; 202), wherein, in an assembled configuration, at least the first pouch (101; 201) and the support member (102; 202) are secured against movement relative to each other,
**characterized in that** at least the first pouch (101; 201) and the support member (102; 202) are secured against movement relative to each other by at least one of the first and second pouches (101, 103; 201, 203) being folded around the support member (102; 202).

2. The packaging assembly of claim 1, wherein the support member (102) is disposed outside the second pouch (203), between the first pouch (101) and the second pouch (103), or inside the first pouch.

3. The packaging assembly of claim 1 or 2, wherein the support member (102) is sized and shaped to support at least the first pouch (101) such that at least a portion of a rim region of the first pouch (101) extends beyond the support member (102), wherein said portion of the rim region is folded around the support member (102).

4. The packaging assembly of claim 3, wherein the first pouch (101) has a substantially rectangular shape, and said portion of the rim region of the first pouch (101) comprises at least one edge (103, 104) of the first pouch (101), preferably at least two opposing edge portions (103, 104) of the first pouch (101).

5. The packaging assembly of claim 1 or 2, wherein the support member (202) is sized and shaped to support at least the second pouch (203), such that at least a portion of a rim region of the second pouch (203), preferably at least two opposing edge portions (213, 214) of the second pouch (203), extends into or beyond a rim region (224, 225, 226, 227), of the support member (202), wherein the rim region (224, 225, 226, 227) of the support member (202) is defined by at least one predetermined fold line (228, 229, 230, 231).

6. The packaging assembly of claim 5, wherein at least a portion (226, 227) of the rim region of the support member (202), preferably at least two opposing edge portions (226, 227) of the support member (202), is folded along the predetermined fold line (230, 231), wherein at least the second pouch (203) is folded along with the support member (202) in order to secure the second pouch (203) relative to the support member (202).

7. A packaging system for a medical device (100; 200), comprising the packaging assembly of any one of claims 1 to 6 and a secondary packaging (118; 218), wherein the secondary packaging (118; 218) is sized and shaped to receive the packaging assembly, wherein inner dimensions of the secondary packaging (118; 218) correspond to outer dimensions of the packaging assembly in order to secure the packaging assembly against relative movement with respect to the secondary packaging (118; 218).

8. A method of packaging a medical device (100; 200), the method comprising the steps of:
a) inserting the medical device (100; 200) into a first pouch (101; 201) and sealing the first pouch (101; 201) to provide a sterile barrier between the medical device (100; 200) and the environment,
b) inserting the first pouch (101; 201) containing the medical device (100; 200) into a second pouch (103; 203)
c) providing at least one support member (102; 202), and
d) folding at least one of the first and second pouches (101, 103; 201, 203) around the support member (102; 202) such that at least the first pouch (101; 201) and the support member (102; 202) are secured against movement relative to each other, wherein steps c) and d) are carried out either before or after step b).

9. The method of claim 8, wherein folding the first pouch (101) around the support member (102) is performed before inserting the first pouch (101) containing the medical device (100) into the second pouch (103), the method preferably further comprising the step of inserting the support member (102) along with the first pouch (101) containing the medical device (100) into the second pouch (103).

10. The method of claim 8 or 9, wherein the support member (102) is sized and shaped to support at least the first pouch (101) such that at least a portion of a rim region of the first pouch (101), preferably at least two opposing edge portions (103, 104) of the first pouch (101), extends beyond the support member (102), wherein the folding step comprises folding said portion of the rim region around the support member (102).

11. The method of claim 8, further comprising the step of placing the second pouch (203) containing the first pouch (201) and the medical device (200) on the support member (202) after sealing the second pouch (203), wherein folding at least one of the first and second pouches (201, 203) around the support member (202) is carried out after placing the second pouch (203) on the support member (202).

12. The method of claim 8 or 11, wherein the support member (202) is sized and shaped to support at least the second pouch (203), such that at least a portion (213, 214) of a rim region of the second pouch (203), preferably at least two opposing edge portions (213, 214) of the second pouch (203), extends into or beyond a rim region (224, 225, 226, 227) of the support member (202), wherein the rim region (224, 225, 226, 227) of the support member (202) is defined by at least one predetermined fold line (228, 229, 230, 231).

13. The method of any one of claims 8, 11 and 12, wherein the folding step comprises folding at least a first portion (224, 225) of the rim region of the support member (202), preferably at least two opposing edge portions (224, 225) of the support member (202), along the predetermined fold line (228, 229).

14. The method of claim 13, wherein the folding step further comprises folding at least a second portion (226, 227) of the rim region of the support member (202), preferably at least two opposing edge portions (226, 227) of the support member (202), along a predetermined fold line (230, 231) in an opposing direction with respect to the first portion (224, 225) of the rim region of the support member (202), wherein at least the second pouch (203) is folded along with the support member (202) in order to secure the second pouch (203) relative to the support member (202).

15. The method of any one of claims 8 to 14, further comprising the step of placing the packaging assembly in a secondary packaging (118; 218), wherein the secondary packaging (118; 218) is sized and shaped to receive the packaging assembly, wherein inner dimensions of the secondary packaging (118; 218) correspond to outer dimensions of the packaging assembly in order to secure the packaging assembly against relative movement with respect to the secondary packaging (118; 218).

## Patentansprüche

1. Verpackungsanordnung für eine medizinische Vorrichtung (100; 200), die zumindest einen ersten Beutel (101; 201) und einen zweiten Beutel (103; 203) umfasst, der versiegelt ist, um eine sterile Barriere zwischen der Umgebung und der medizinischen Vorrichtung (100; 200) bereitzustellen, die im ersten Beutel (101; 201) aufgenommen ist, der wiederum im zweiten Beutel (103; 203) aufgenommen ist, wobei die Verpackungsanordnung ferner zumindest ein Tragelement (102; 202) umfasst, wobei in einer zusammengebauten Konfiguration zumindest der erste Beutel (101; 201) und das Tragelement (102; 202) gegen eine Bewegung relativ zueinander gesichert sind,
**dadurch gekennzeichnet, dass** zumindest der erste Beutel (101; 201) und das Tragelement (102; 202) gegen eine Bewegung relativ zueinander durch zumindest einen der um das Tragelement (102; 202) gefalteten ersten und zweiten Beutel (101, 103; 201, 203) gesichert sind.

2. Verpackungsanordnung nach Anspruch 1, wobei das Tragelement (102) außerhalb des zweiten Beutels (203), zwischen dem ersten Beutel (101) und dem zweiten Beutel (103), oder innerhalb des ersten Beutels angeordnet ist.

3. Verpackungsanordnung nach Anspruch 1 oder 2, wobei das Tragelement (102) zum Abstützen zumindest des ersten Beutels (101) bemessen und geformt ist, sodass sich zumindest ein Bereich eines Randabschnitts des ersten Beutels (101) über das Stützelement (102) hinaus erstreckt, wobei der Bereich des Randabschnitts um das Tragelement (102) gefaltet ist.

4. Verpackungsanordnung nach Anspruch 3, wobei der erste Beutel (101) eine im Wesentlichen rechteckige Form aufweist und der Bereich des Randabschnitts des ersten Beutels (101) zumindest einen Rand (103, 404) des ersten Beutels (101), vorzugsweise zumindest zwei gegenüberliegende Randabschnitte (103, 404) des ersten Beutels (101) umfasst.

5. Verpackungsanordnung nach Anspruch 1 oder 2, wobei das Tragelement (202) zum Abstützen zumindest des zweiten Beutels (203) bemessen und geformt ist, sodass sich zumindest ein Bereich eines Randabschnitts des zweiten Beutels (203), vorzugsweise zumindest zwei gegenüberliegende Randabschnitte (213, 214) des zweiten Beutels (203), in oder über einen Randabschnitt (224, 225, 226, 227) des Tragelements (202) hinaus erstrecken, wobei der Randabschnitt (224, 225, 226, 227) des Tragelements (202) durch zumindest eine vorbestimmte Faltlinie (228, 229, 230, 231) definiert ist.

6. Verpackungsanordnung nach Anspruch 5, wobei zumindest ein Bereich (226, 227) des Randabschnitts des Tragelements (202), vorzugsweise zumindest zwei gegenüberliegende Randbereiche (226, 227) des Tragelements (202), entlang der vorbestimmten Faltlinie ((230, 231) gefaltet ist, wobei zumindest der zweite Beutel (203) zusammen mit dem Tragelement (202) zum Sichern des zweiten Beutels (203) relativ zum Tragelement (202) gefaltet ist.

7. Verpackungssystem für eine medizinische Vorrichtung (100; 200), welche die Verpackungsanordnung nach einem der Ansprüche 1 bis 6 und eine Sekundärverpackung (118; 218) umfasst, wobei die Sekundärverpackung (118; 218) zum Aufnehmen der Verpackungsanordnung bemessen und geformt ist, wobei die Innenabmessungen der Sekundärverpackung (118; 218) den Außenabmessungen der Verpackungsanordnung entsprechen, um die Verpackungsanordnung gegen eine Relativbewegung in Bezug auf die Sekundärverpackung (118; 218) zu sichern.

8. Verfahren zum Verpacken einer medizinischen Vorrichtung (100; 200), wobei das Verfahren die folgenden Schritte umfasst:
a Einführen der medizinischen Vorrichtung (100; 200) in einen ersten Beutel (101; 201) und Verschließen des ersten Beutels (101; 201), um eine sterile Barriere zwischen der medizinischen Vorrichtung (100; 200) und der Umgebung bereitzustellen,
b Einführen des ersten Beutels (101; 201), der die medizinische Vorrichtung (100; 200) enthält, in einen zweiten Beutel (103; 203),
c Bereitstellen zumindest eines Tragelements (102; 202), und
d Falten zumindest eines der ersten und zweiten Beutel (101, 103; 201, 203), um das Tragelement (102; 202), sodass zumindest der erste Beutel (101; 201) und das Tragelement (102; 202) gegen eine Bewegung relativ zueinander gesichert sind, wobei die Schritte c) und d) entweder vor oder nach dem Schritt b) durchgeführt werden.

9. Verfahren nach Anspruch 8, wobei das Falten des ersten Beutels (101) um das Tragelement (102) vor dem Einführen des ersten Beutels (101), der die medizinische Vorrichtung (100) enthält, in den zweiten Beutel (103) durchgeführt wird, wobei das Verfahren vorzugsweise ferner den Schritt des Einführens des Tragelements (102) zusammen mit dem ersten Beutel (101), der die medizinische Vorrichtung (100) enthält, in den zweiten Beutel (103) umfasst.

10. Verfahren nach Anspruch 8 oder 9, wobei das Tragelement (102) zum Abstützen zumindest des ersten Beutels (101) bemessen und geformt ist, sodass sich zumindest ein Bereich eines Randabschnitts des ersten Beutels (101), vorzugsweise zumindest zwei gegenüberliegende Randabschnitte (103, 104) des ersten Beutels (101), über das Tragelement (102) hinaus erstrecken, wobei der Faltschritt das Falten des Bereichs des Randabschnitts um das Tragelement (102) umfasst.

11. Verfahren nach Anspruch 8, das ferner den Schritt des Platzierens des zweiten Beutels (203), der den ersten Beutel (201) und die medizinische Vorrichtung (200) enthält, auf dem Tragelement (202) nach dem Verschließen des zweiten Beutels (203) umfasst, wobei das Falten zumindest eines der ersten und zweiten Beutel (201, 203) um das Tragelement (202) nach dem Platzieren des zweiten Beutels (203) auf dem Tragelement (202) ausgeführt wird.

12. Verfahren nach Anspruch 8 oder 11, wobei das Tragelement (202) zum Abstützen zumindest des zweiten Beutels (203) bemessen und geformt ist, sodass sich zumindest ein Bereich (213, 214) eines Randabschnitts des zweiten Beutels (203), vorzugsweise zumindest zwei gegenüberliegende Randabschnitte (213, 214) des zweiten Beutels (203), in oder über einen Randabschnitt (224, 225, 226, 227) des Tragelements (202) hinaus erstrecken, wobei der Randabschnitt (224, 225, 226, 227) des Tragelements (202) durch zumindest eine vorbestimmte Faltlinie (228, 229, 230, 231) definiert wird.

13. Verfahren nach einem der Ansprüche 8, 11 und 12, wobei der Faltschritt das Falten zumindest eines ersten Bereichs (224, 225) des Randabschnitts des Tragelements (202), vorzugsweise zumindest zweier gegenüberliegender Randbereiche (224, 225) des Tragelements (202), entlang der vorbestimmten Faltlinie (228, 229) umfasst.

14. Verfahren nach Anspruch 13, wobei der Faltschritt ferner das Falten zumindest eines zweiten Bereichs (226, 227) des Randabschnitts des Tragelements (202), vorzugsweise zumindest zweier gegenüberliegender Randbereiche (226, 227) des Tragelements (202), entlang einer vorbestimmten Faltlinie (230, 231) in entgegengesetzter Richtung in Bezug auf den ersten Abschnitt (224, 225) des Randabschnitts des Tragelements (202) umfasst, wobei zumindest der zweite Beutel (203) zusammen mit dem Tragelement (202) gefaltet wird, um den zweiten Beutel (203) relativ zum Tragelement (202) zu sichern.

15. Verfahren nach einem der Ansprüche 8 bis 14, das ferner den Schritt des Platzierens der Verpackungsanordnung in einer Sekundärverpackung (118; 218) umfasst, wobei die Sekundärverpackung (118; 218) zum Aufnehmen der Verpackungsanordnung bemessen und geformt ist, wobei die Innenabmessungen der Sekundärverpackung (118; 218) den Außenabmessungen der Verpackungsanordnung entsprechen, um die Verpackungsanordnung gegen eine Relativbewegung in Bezug auf die Sekundärverpackung (118; 218) zu sichern.

## Revendications

1. Ensemble de conditionnement pour un dispositif médical (100; 200), comprenant au moins un premier sachet (101; 201) et un second sachet (103; 203) scellés de manière à fournir une barrière stérile entre l'environnement et le dispositif médical (100; 200) reçu dans le premier sachet (101; 201), lequel à son tour est reçu dans le second sachet (103; 203) l'ensemble de conditionnement comprenant en outre au moins un élément de support (102; 202), dans lequel dans une configuration assemblée, au moins le premier sachet (101; 201) et l'élément de support (102; 202) sont arrimés contre tout mouvement l'un par rapport à l'autre,
**caractérisé en ce qu'**au moins le premier sachet (101; 201) et l'élément de support (102; 202) sont arrimés contre tout mouvement l'un par rapport à l'autre par au moins un sachet parmi les premier et second sachets (101, 103; 201, 203) étant repliés autour de l'élément de support (102; 202).

2. Ensemble de conditionnement selon la revendication 1, dans lequel l'élément de support (102) est disposé à l'extérieur du second sachet (203), entre le premier sachet (101) et le second sachet (103), ou à l'intérieur du premier sachet.

3. Ensemble de conditionnement selon la revendication 1 ou 2, dans lequel l'élément de support (102) est dimensionné et façonné de manière à supporter au moins le premier sachet (101) de telle sorte qu'au moins une portion d'une région de rebord du premier sachet (101) s'étende au-delà de l'élément de support (102), et dans lequel ladite portion de la région de rebord est repliée autour de l'élément de support (102).

4. Ensemble de conditionnement selon la revendication 3, dans lequel le premier sachet (101) présente une forme en grande partie rectangulaire, et ladite portion de la région de rebord du premier sachet (101) comprend au moins un bord (103, 104) du premier sachet (101), de préférence au moins deux portions de bord opposées (103, 204) du premier sachet (101).

5. Ensemble de conditionnement selon la revendication 1 ou 2, dans lequel l'élément de support (202) est dimensionné et façonné de manière à supporter au moins le second sachet (203) de telle sorte qu'au moins une portion d'une région de rebord du second sachet (203), de préférence au moins deux portions de bord opposées (213, 214) du second sachet (203), s'étendent jusque dans une région de rebord (224, 225, 226, 227) de l'élément de support (202), ou au-delà de celle-ci, dans lequel la région de rebord (224, 225, 226, 227) de l'élément de support (202) est définie par au moins une ligne de pliage prédéterminée (228, 229, 230, 231).

6. Ensemble de conditionnement selon la revendication 5, dans lequel au moins une portion (226, 227) de la région de rebord de l'élément de support (202), de préférence au moins deux portions de bord opposées (226, 227) de l'élément de support (202), est repliée le long de la ligne de pliage prédéterminée (230, 231), et dans lequel au moins le second sachet (203) est replié conjointement avec l'élément de support (202) afin d'arrimer le second sachet (203) par rapport à l'élément de support (202).

7. Système de conditionnement pour un dispositif médical (100; 200), comprenant l'ensemble de conditionnement selon l'une quelconque des revendications 1 à 6 et un emballage secondaire (118; 218), dans lequel l'emballage secondaire (118; 218) est dimensionné et façonné pour recevoir l'emballage secondaire ; dans lequel les dimensions intérieures de l'emballage secondaire (118; 218) correspondent aux dimensions extérieures de l'ensemble de conditionnement afin d'arrimer l'ensemble de conditionnement contre tout mouvement par rapport à l'emballage secondaire (118; 218).

8. Procédé destiné à conditionner un dispositif médical (100; 200), le procédé comprenant les étapes consistant à :
a) introduire le dispositif médical (100; 200) dans un premier sachet (101; 201) et sceller le premier sachet (101; 201), afin de fournir une barrière stérile entre le dispositif médical (100; 200) et l'environnement ;
b) introduire le premier sachet (101; 201) contenant le dispositif médical (100; 200) dans un second sachet (103; 203) ;
c) fournir au moins un élément de support (102; 202), et
d) replier au moins un sachet parmi les premier et second sachets (101, 103; 201, 203) autour de l'élément de support (102; 202), de telle sorte qu'au moins le premier sachet (101; 201) et l'élément de support (102; 202) sont arrimés contre tout mouvement l'un par rapport à l'autre, et dans lequel les étapes c) et d) sont exécutées soit avant, soit après l'étape b).

9. Procédé selon la revendication 8, dans lequel l'étape pour replier le premier sachet (101) autour de l'élément de support (102) est réalisée avant d'introduire le premier sachet (101) contenant le dispositif médical (100) dans le second sachet (103), le procédé comprenant, en outre et de préférence, l'étape pour introduire l'élément de support (102) conjointement avec le premier sachet (101) contenant le dispositif médical (100) dans le second sachet (103).

10. Procédé selon la revendication 8 ou 9, dans lequel l'élément de support (102) est dimensionné et façonné de manière à supporter au moins le premier sachet (101) de telle sorte qu'au moins une portion d'une région de rebord du premier sachet (101), de préférence au moins deux portions de bord opposées (103, 104) du premier sachet (101), s'étende au-delà de l'élément de support (102), et dans lequel l'étape pour replier comprend l'étape pour replier ladite portion de la région de rebord autour de l'élément de support (102).

11. Procédé selon la revendication 8, comprenant en outre l'étape pour placer le second sachet (203) contenant le premier sachet (201) et le dispositif médical (200) sur l'élément de support (202) après avoir scellé le second sachet (203), et dans lequel l'étape pour replier au moins un sachet parmi les premier et second sachets (201, 203) autour de l'élément de support (202) est réalisée après avoir placé le second sachet (203) sur l'élément de support (202).

12. Procédé selon la revendication 8 ou 11, dans lequel l'élément de support (202) est dimensionné et façonné de manière à supporter au moins le second sachet (203) de telle sorte qu'au moins une portion (213, 214) d'une région de rebord du second sachet (203), de préférence au moins deux portions de bord opposées (213, 214) du second sachet (203), s'étende jusque dans une région de rebord (224, 225, 226, 227) de l'élément de support (202), ou au-delà de celle-ci, dans lequel la région de rebord (224, 225, 226, 227) de l'élément de support (202) est définie par au moins une ligne de pliage prédéterminée (228, 229, 230, 231).

13. Procédé selon l'une quelconque des revendications 8, 11 et 12, dans lequel l'étape pour replier comprend l'étape pour replier au moins une première portion (224, 225) de la région de rebord de l'élément de support (202), de préférence au moins deux portions de bord opposées (224, 225) de l'élément de support (202), le long de la ligne de pliage prédéterminée (228, 229).

14. Procédé selon la revendication 13, dans lequel l'étape pour replier comprend en outre l'étape pour replier au moins une seconde portion (226, 227) de la région de rebord de l'élément de support (202), de préférence au moins deux portions de bord opposées (226, 227) de l'élément de support (202), le long d'une ligne de pliage prédéterminée (230, 231) dans une direction opposée par rapport à la première portion (224, 225) de la région de rebord de l'élément de support (202), et dans lequel au moins le second sachet (203) est replié conjointement avec l'élément de support (202) afin d'arrimer le second sachet (203) par rapport à l'élément de support (202).

15. Procédé selon l'une quelconque des revendications 8 à 14, comprenant en outre l'étape pour placer l'ensemble de conditionnement dans un emballage secondaire (118 ; 218) ; dans lequel l'emballage secondaire (118 ; 218) est dimensionné et façonné pour recevoir l'ensemble de conditionnement ; dans lequel les dimensions intérieures de l'emballage secondaire (118; 218) correspondent aux dimensions extérieures de l'ensemble de conditionnement afin d'arrimer l'ensemble de conditionnement contre tout mouvement par rapport à l'emballage secondaire (118; 218).
